# EUROPEAN PATENT APPLICATION

(11) **EP 2 628 446 A1**
(43) Date of publication of application: **21.08.2013**
(21) Application number: 10858404.6
(22) Date of filing: 14.10.2010
(51) Int. Cl.: A61B 5/1455

(54) **EQUIPMENT FOR IN VIVO DATA ACQUISITION AND ANALYSIS**

(71) Applicant: Hitachi, Ltd., Chiyoda-ku Tokyo 100-8280 (JP)
(72) Inventor: KAWAHARA, Takayuki, Tokyo 100-8220 (JP); TAKEMURA, Riichiro, Tokyo 100-8220 (JP); SONEHARA, Tsuyoshi, Tokyo 100-8220 (JP); NAGASAKA, Akio, Tokyo 100-8220 (JP)
(74) Representative: MERH-IP Matias Erny Reichl Hoffmann
(86) International application number: PCT/JP2010/068054
(87) International publication number: WO 2012/049753

(57) **Abstract**

To obtain a blood sugar level accurately, the location of a blood-vessel part is specified by using a first wavelength at which absorption by hemoglobin, which is a component unique to blood, is high, and data of light absorbance measured by using a second wavelength at which absorption by glucose is high is separated into a blood-vessel part and other parts.

## Description

### TECHNICAL FIELD

The present invention relates to a device that non-invasively acquires biological information and analyzing the biological information using light sources of two types of wavelengths and a photosensor array.

### BACKGROUND ART

The social needs for a desire to easily understand daily health status have been increasing. Among barometers to understand daily health status, the blood sugar level (glucose concentration in blood) is one of the barometers for finding out the state of health, and not only the people who suffer diabetes but also many people are interested in that. Therefore, development for finding out the blood sugar level in a body fluid by using near-infrared spectroscopy has been underway as described below. It is because such biological information can be non-invasively obtained when near-infrared spectroscopy is used.

Patent Literature 1 discloses that white light is caused to enter a living body by a spot, the light that has passed therethrough is collected by the spot (or collected by a plurality of spots), the light is caused to be two-dimensionally spread light by a strip-like optional wavelength selecting filter, and the intensity information thereof is obtained at one time by a two-dimensional image sensor. As the two-dimensional image sensor, an example of InGaAs-CCD light-receiving elements is shown. The light that has passed through the optional wavelength selecting filter is divided into two by a mirror, one of them is detected as a light-receiving level of an optical signal separated by wavelengths in a range of 1200 nm to 1600 nm by the InGaAs-CCD light-receiving elements, and the other is detected as a light-receiving level of an optical signal separated by wavelengths in a range of 400 nm to 1100 nm by different CCD light-receiving elements. In this manner, this is a device that analyzes, at one time, the biological information obtained at spots by using the arbitrary wavelength select filer and the two-dimensional image sensor.

According to Patent Literature 2, this is a device for finding a tumor (s) ; and it discloses a one-dimensional (or spot) prober that uses a first wavelength that glucose absorbs and a second wavelength that water absorbs and measures the outgoing light from a living body at the respective wavelengths. This is moved above a living body to specify a part where a portion having a high glucose concentration and a region having a high water concentration are alternately shown.

Patent Literature 3 describes techniques in which a first wavelength that is largely absorbed by glucose and a second wavelength that is absorbed a little by water molecules and hemoglobin are used, and a baseline of data by the first wavelength is corrected by using spectra of the second wavelength.

Patent Literature 4 discloses a method of checking the concentration of glucose by checking the absorption that is caused by OH groups, CH groups, and NH groups of glucose molecules by three wavelengths.

Patent Literature 5 discloses a device in which light is caused to enter the inner side of a flexing portion of a finger joint having blood vessels concentrated near the skin in order to find out the components in blood, the outgoing light largely including a part that has passed through the blood vessels is measured regarding two wavelengths, i.e., a first wavelength at which absorption by glucose is high and a second wavelength not absorbed by glucose, and the concentration of glucose is checked by the ratio of the two. A detector is an array, and it discloses that the concentration of glucose is checked by obtaining the ratio between an output at which absorption is highest and selected from the output of the first wavelength and an output of the second wavelength at the same location.

### Citation List

### Patent Literatures

Patent Literature 1: Japanese Patent Application Laid-Open Publication No. 2004-252214
Patent Literature 2: Japanese Patent Application Laid-Open Publication No. 2007-26784
Patent Literature 3: Japanese Patent Application Laid-Open Publication No. 2004-257835
Patent Literature 4: Japanese Patent Application Laid-Open Publication No. H10-325794
Patent Literature 5: Japanese Patent Application Laid-Open Publication No. H11-137538

### DISCLOSURE OF THE INVENTION

### PROBLEMS TO BE SOLVED BY THE INVENTION

Since the blood sugar level is the concentration of glucose, the glucose concentration in blood has to be measured. However, in measurement using near-infrared spectroscopy, a glucose absorbing wavelength region and a water wavelength region are overlapped; therefore, near-infrared spectroscopy and analysis focusing on a blood-vessel part(s) are impossible.

### MEANS FOR SOLVING THE PROBLEMS

A typical means described in the present invention includes: a first light source that radiates light having a first wavelength; a second light source that radiates light having a second wavelength; a photosensor array that detects outgoing light from a living body irradiated with the light from the first light source and the second light source; and an analyzer that analyzes data from the photosensor array; in the means, the analyzer specifies a region, as a blood-vessel part, in which the intensity of the outgoing light from the living body irradiated with the light having the first wavelength exceeds a predetermined threshold value, separates the data based on the intensity of the outgoing light from the living body irradiated with the light having the second wavelength into data of the specified region and data of other regions, and subjects the data to analysis.

The inner side of a finger, a palm, the back of a hand, a lip, etc. can be used as the living body.

To realize the photosensor array, other than photodiodes using InGaAs, a photosensor array is formed at a lower price by photodiodes using SiGe in which Ge is grown on a Si substrate.

### EFFECTS OF THE INVENTION

A device capable of acquiring biological information of blood at high accuracy and precisely analyzing, for example, the concentration of glucose can be realized. By virtue of this, the blood sugar level (the concentration of glucose in blood) can be non-invasively obtained.

### BRIEF DESCRIPTIONS OF THE DRAWINGS

FIG. 1A is an image formation of a case in which a finger is irradiated with light having a wavelength A;
FIG. 1B is an image formation of a case in which the finger is irradiated with light having a wavelength B;
FIG. 2 is a circuit diagram of a photosensor cell;
FIG. 3 is a device configuration example of the photosensor cell;
FIG. 4 is a block diagram of a photosensor array chip;
FIG. 5 is a block diagram of a device for acquiring and analyzing biological information;
FIG. 6 is a diagram illustrating a flow of acquiring biological information;
FIG. 7A is a diagram illustrating light absorbance in a case in which irradiation of light having the wavelength A is carried out;
FIG. 7B is a diagram illustrating light absorbance in a case in which irradiation of light having the wavelength B is carried out;
FIG. 8A is a data string acquired by irradiation of light having the wavelength A;
FIG. 8B is a data string acquired by irradiation of light having the wavelength B;
FIG. 8C is a data string of the wavelength B extracted by using the data string of the wavelength A;
FIG. 9 is a diagram illustrating light absorbance characteristics of hemoglobin with respect to wavelengths;
FIG. 10A illustrates near-infrared spectra of glucose and quadratic differentials thereof;
FIG. 10B illustrates near-infrared spectra of water and quadratic differentials thereof;
FIG. 11 is a diagram illustrating another configuration example of the photosensor array;
FIG. 12 is a device configuration example of the photosensor array of FIG. 11;
FIG. 13 is a diagram illustrating sensitivity characteristics of photosensors with respect to wavelengths;
FIG. 14A is a block diagram of a device for acquiring and analyzing biological information;
FIG. 14B is a diagram illustrating an operation example the device;
FIG. 15 is a diagram for explaining a relation between a diameter of a blood vessel and a size of a photosensor cell;
FIG. 16 is a diagram illustrating wavelength dependency of the light absorbance of a substance;
FIG. 17A is a block diagram of a device for acquiring and analyzing biological information;
FIG. 17B is a plan view of a filter plate thereof;
FIG. 18 is a schematic diagram of a photosensor array provided with filters;
FIG. 19 is a block diagram showing a configuration of a lateral-surface irradiation system of a device for acquiring and analyzing biological information;
FIG. 20 is a block diagram showing a configuration of a lateral-surface irradiation system of a device for acquiring and analyzing biological information;
FIG. 21A is a block diagram of a device for acquiring and analyzing biological information;
FIG. 21B and 21C are drawings showing operation principles of the device for acquiring and analyzing biological information;
FIGS. 22A to 22C are block diagrams of a device for acquiring and analyzing biological information; and
FIG. 23 is a diagram illustrating a state of scanning a finger by an array sensor of the device for acquiring and analyzing biological information.

### BEST MODE FOR CARRYING OUT THE INVENTION

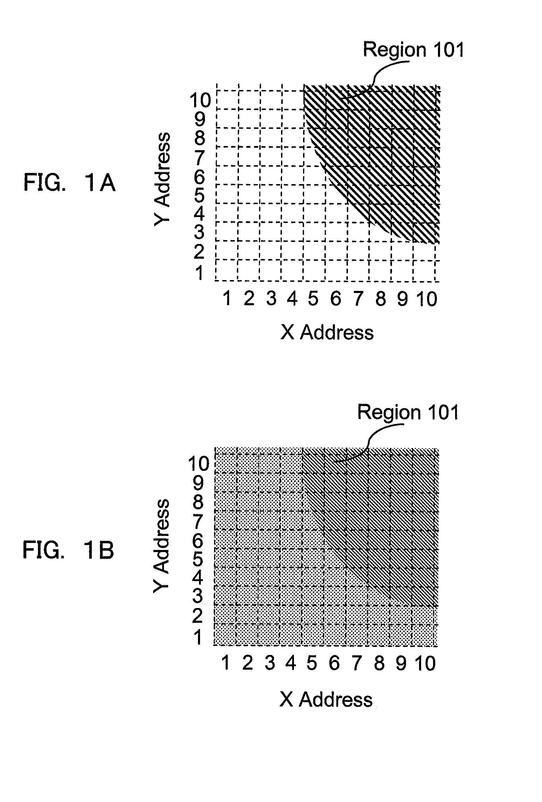

In the present invention, a living body is irradiated with light (having a specific wavelength or white light), and the light transmitted through an internal part of the living body is caused to form an image on a photosensor array to acquire biological information non-invasively. FIG. 1A illustrates image formation in a case in which a finger is irradiated with light having a wavelength A, and FIG. 1B illustrates image formation in a case in which a finger is irradiated with light having a wavelength B.

Each of matrixes (broken lines) schematically represents the photosensor array, which is two-dimensionally filled with cells of photosensors, and each section of the matrix represents a photosensor cell. Each of the cells can be specified by a horizontal number (X address) and a vertical number (Y address) given to the matrix. When a wavelength from 810 to 940 nm, for example, 860 nm is used as the wavelength A, as shown in FIG. 1A, a shaded region 101 is shown in the upper right of the matrix (the region 101 is darker than the surrounding area thereof). This is an image of a vein(s) present in the inner side of the finger. Since the light in the vicinity of the wavelength of 860 nm is strongly absorbed by hemoglobin, this region becomes darker than the surrounding area thereof. Light intensity data (or data representing the intensity of light absorption) is collected from the photosensor cells. Since hemoglobin is present only in blood vessels, the absorption intensity thereof is significantly different in blood-vessel parts and other parts. Therefore, dark cells (representing blood-vessel parts) and bright cells (representing parts other than the blood-vessel parts) can be clearly distinguished from each other by setting an appropriate threshold value for the output of the photosensor cells.

Next, FIG. 1B illustrates a result of irradiating the same measurement site with light within a range of 1500 to 1700 nm as the wavelength B and forming an image on the photosensor array. This wavelength is a wavelength that is absorbed by glucose, but is also absorbed by water. However, glucose is mainly present in blood vessels while water is substantially uniformly distributed in the entire body. Therefore, in the image formation by the wavelength B, the shaded region 101 is expressed in the upper right of the matrix as well as FIG. 1A due to the presence of glucose. However, the contrast between the region 101 and the other region is significantly small as compared to that in FIG. 1A. Therefore, it is difficult to specify the part of blood vessels by the measurement of only FIG. 1B, since there is also influence of noise, etc. inevitable in actual measurement. On the other hand, in the present invention, the position of the blood vessel (region 101) can be specified by the X address and the Y address of the photosensor array by measurement at the wavelength A. The data of the photosensor cells measured at the wavelength B can be separated into the data of a blood-vessel part and the data of other parts by using the addresses of the region 101 specified by the measurement by the wavelength A. In other words, only the data of the blood-vessel part can be selected from the measurement site.

Since the blood-vessel part can be specified in this manner, time-dependent changes of the ratio of water and glucose at the blood-vessel part can be obtained from changes of the absorbance at the part. Upon calculation of the ratio of water and glucose at the blood-vessel part, accuracy can be enhanced by carrying out the calculation based on the absorbance of the part other than the blood-vessel part. More specifically, since the absorbance of light is affected by the body temperature of a living body, the absorbance of light thus has a different value depending on the body temperature of the day even at the same blood sugar level. However, the absorbance at the blood-vessel part mainly depending on water and glucose and the absorbance at the part other than blood vessels mainly depending on water are measured at the same body temperature; therefore, the concentration of glucose in blood can be obtained with high accuracy by obtaining the difference between the data of the blood-vessel part and the data of the part other than vessels.

In a case in which the finger is irradiated with light from the upper side or lateral face thereof and the outgoing light from the inner side is measured, the light that transmits through a deep part of the finger is diffused in the body and is not expressed as meaningful information. Only the light that has transmitted through and diffused in a shallow part close to the surface of the skin is detected on the photosensor array as meaningful information. Therefore, the detected blood-vessel part is only a vein(s) present in a part close to the skin of the finger. In this manner, the information about the blood sugar level can be obtained by comparing the states of the outgoing light from the vein in the vicinity of the surface of the finger and the outgoing light from the part other than that.

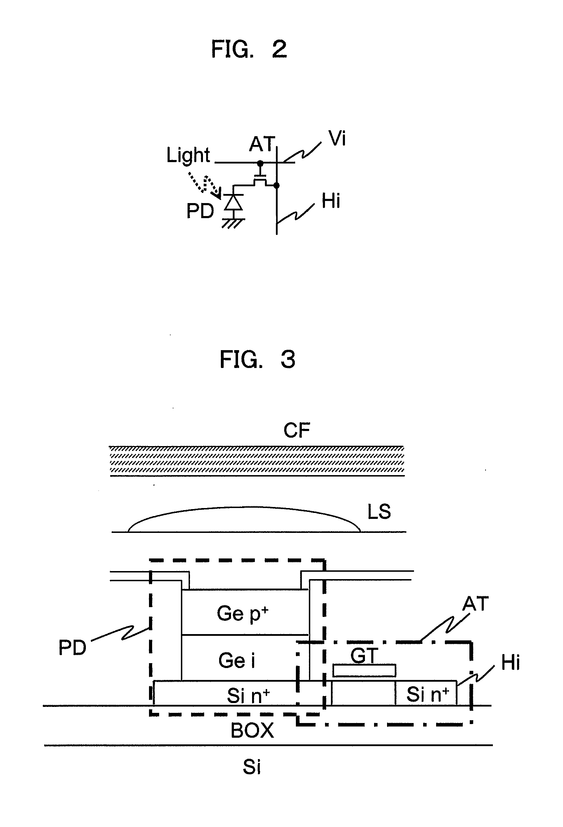

FIG. 2 is a circuit diagram of the photosensor cell which composes the photosensor array. The cell is provided at an intersecting point of a row signal line Vi and a column signal line Hj, has a photodiode PD having an anode connected to a reference (ground) potential and a source-drain path between a cathode of the photodiode PD and the column signal line Hj, and has an access transistor AT, of which a gate is controlled by the row signal line Vi. The access transistor AT is not limited to but composed of a MOS transistor. As a simplest example, for example, the photodiode PD can be composed of a reverse-direction pn-junction diode, which is formed by providing an n⁺-type impurity region (cathode) in a p-type semiconductor substrate (anode). In this case, at the photodiode PD, entered light (photons) generates electron-hole pairs having the energy that is capable of jumping over the band gap of Si. A current or accumulated voltage caused by this is extracted by controlling a gate voltage of the access transistor AT. Note that the structure of the cell is not limited to the simple structure described above. The structures of various known photodiodes that provide an amplifying action by receiving the electric potential of the n⁺-region by the gate of a MOS transistor can be used.

As described above, in the present invention, infrared light having a comparatively long wavelength such as 1500 to 1700 nm as the wavelength B is used. It is difficult for the energy of the light having such a long wavelength to generate electron-hole pairs that have the energy for jumping over the band gap of Si. Therefore, the photodiode PD is preferable to be composed of a semiconductor having a smaller band gap. As the semiconductor having a small band gap, a compound semiconductor such as InGaAs can be used. On the other hand, a photosensor cell that non-invasively obtains the blood sugar level for daily healthcare is preferred to be fabricated at a low cost. FIG. 3 schematically illustrates a device cross section of a photosensor cell suitable for reducing cost. An oxide film BOX is formed on a Si substrate, and a Si layer is further grown thereon, and this is what they call an SOI structure. In this case, in the part of the photodiode PD (in the frame of a broken line), a non-doped Ge i layer and a p-type doped Ge p⁺ layer are sequentially stacked on a Si n⁺ layer, and the Ge p⁺ layer is grounded, which is not shown. A gate GT (corresponding to the row signal line Vi) is formed on a channel layer between the Si n⁺ layer of the photodiode PD and the column signal line Hi via a gate insulating film, thereby forming the access transistor AT. In this structure, electron-hole pairs can be generated by the energy of the light having a comparatively long wavelength such as 1500 to 1700 nm, and light can be detected at a low cost. Furthermore, a condensing lens LS is fabricated in an upper layer of an opening of the photodiode PD. This can be fabricated for each photosensor by utilizing the structure of SiO₂. By virtue of this, light can be collected from an area larger than that of the opening of the photodiode PD. In this example, a color filter CF is further disposed at an upper part of that. Even in a case in which the light having a desired wavelength cannot be obtained only with a light source, data of the light having a necessary wavelength can be extracted by using the color filter CF. Furthermore, a filter that allows passage of the wavelength(s) that is different for each photosensor cell may be mounted as the color filter CF. In this manner, data of a plurality of wavelengths can be obtained by one time of measurement. Therefore, if the frequency dependency of the light absorbance of the substance serving as a target to be measured is characteristic, the data with which a highly accurate concentration can be calculated can be obtained.

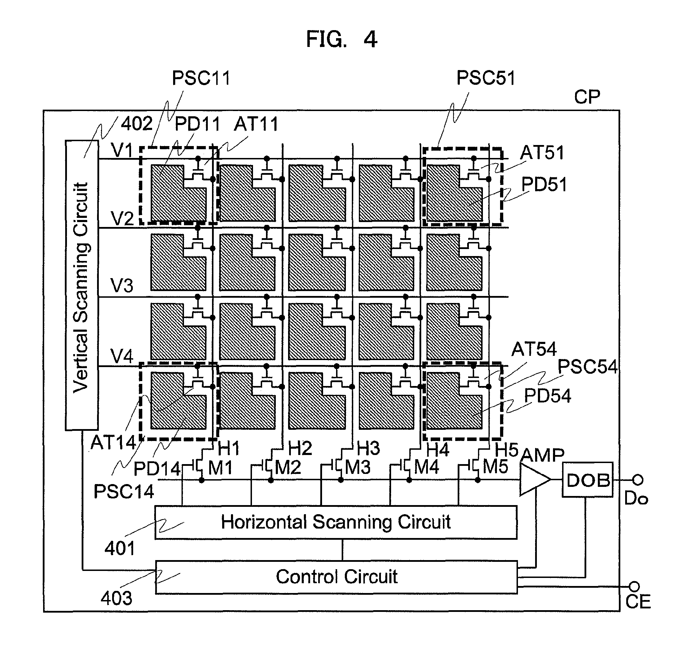

FIG. 4 is an embodiment describing a configuration of a photosensor array chip CP of the present invention. On the chip CP, a two-dimensionally spread photosensor array, a horizontal scanning circuit 401 for selectively controlling that, a vertical scanning circuit 402, an amplifier circuit AMP which amplifies signals from the photosensor array, an output buffer DOB, and a control circuit 403 thereof are disposed. A signal that activates and controls the chip CP is represented by and denoted as an activation control signal CE herein. "Do" represents an output terminal (in many cases, a plurality of output terminals are provided). Each of photosensor cells PSC is composed of a photodiode PD (in this case, illustrated as a layout image) and an access transistor AT as explained in FIG. 2. Only four-corner elements are denoted by symbols; however, the photodiodes PD11 to PD54 and the access transistors AT11 to AT54 are two-dimensionally diffused like an array, and the intensity of light at each location thereof is converted to an electric signal.

Gates of the access transistors AT11 to AT54 are connected to row signal lines V1 to V4, and they are controlled by the vertical scanning circuit 402. Therefore, when a desired signal line is selected from among the row signal lines V1 to V4, the access transistors AT are turned on. As a result, the signals detected by the photodiodes PD are output to the column signal lines H1 to H5. The column signal lines H1 to H5 are selectively connected by an input line of the amplifier circuit AMP and column-direction select transistors M1 to M5. This selective connection is controlled by the horizontal scanning circuit 401. For example, when the select transistor M1 is selected by the horizontal scanning circuit 401, the signal of the column signal line H1 is transmitted to the amplifier circuit AMP, and an amplified signal thereof is output to the output Do of the chip via the output buffer DOB.

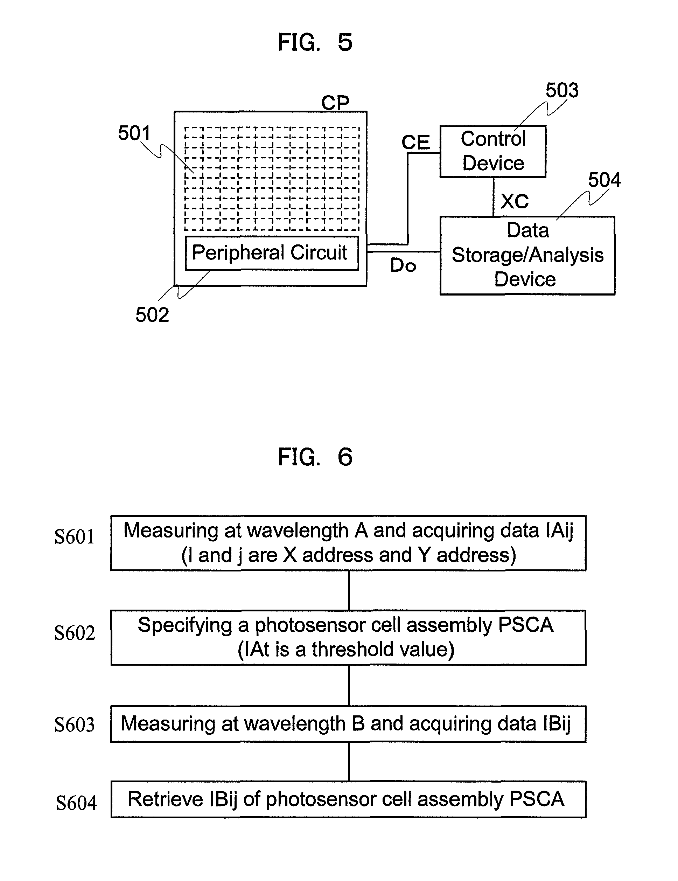

FIG. 5 shows devices connected to a photosensor array chip CP. The chip CP is equivalent to that of FIG. 4 and has a photosensor array 501 and a peripheral circuit 502 (indicating a circuit part other than the sensor array of FIG. 4). A control device 503 carries out activation of the photosensor array chip CP, scanning of data, output of data to the output terminal Do, etc. in accordance with control signals represented by the activation control signal CE. Although not illustrated, the control device 503 also carries out control of a light source for irradiation of a living body. A data saving and analyzing device 504 saves and analyzes data that is from the photosensor array chip CP. The device 504 is controlled also in accordance with control signals XC from the control device; and, in a manner explained in relation to FIG. 1, the device stores data measured at two wavelengths, extracts measurement data of the second wavelength at a location including meaningful information on the photosensor array specified by the first wavelength, or determines the concentration of glucose by comparison with data prepared in advance. As an example, correlations between data obtained by directly collecting blood and measuring the concentration of glucose in the blood and data of the photosensors measured at the same time are stored as a table in the data saving and analyzing device 504 in advance. The concentration of glucose is calculated with, in accordance with the data measured by the photosensor array chip CP, referencing the correlation data. The control device 503 and the data saving and analyzing device 504 are prepared as dedicated devices in some cases, or part thereof may be carried out by a computer (PC). For example, the entirety of the data saving and analyzing device 504 can be achieved by a PC and software installed therein. In that case, transmissions of control signals, etc. can be carried out via, for example, USB terminals.

FIG. 6 illustrates a flow for obtaining data of outgoing light from a region of a part corresponding to a blood vessel(s). This is a procedure for extracting measurement data of the second wavelength B at the location including meaningful information on the photosensor array specified by the first wavelength A.

First, the intensity IA of the outgoing light from a living body is measured by the photosensor array using the wavelength A. The intensity data of the light output from a photosensor cell PSCij having an X address of "i" and a Y address of "j" will be described as IAij. The intensity data IAij is acquired for all of the photosensor cells composing the photosensor array (S601). Next, the magnitude relation of the intensity data IAij and a threshold value IAt determined in advance is subjected to comparison. A gathering of the photosensor cells which have output IAij having a value larger than the threshold value IAt is obtained. The gathering of such photosensor cells is referred to as PSCA and specified by addresses (i, j) (S602). Next, measurement is carried out at the wavelength B, and intensity data IBij of this process is acquired (S603). At the end, IBij of the photosensor cells belonging to PSCA is extracted (S604). A wavelength at which absorption by hemoglobin, which is a component unique to blood, is high is used as the wavelength A, and a wavelength at which absorption by glucose is high is used as the wavelength B; as a result, data containing the intensity data of glucose only from a blood-vessel part can be obtained.

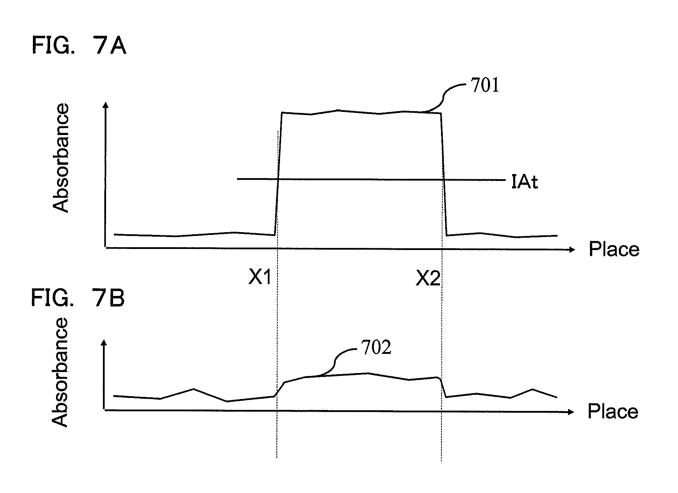

FIGS. 7A to 7B schematically illustrate light absorbance expressed at corresponding locations, both of horizontal axes mean the locations along a certain direction, and vertical axes mean the magnitude of the light absorbance. FIG. 7A is a schematic diagram of an image by the wavelength A. Since the wavelength A is a wavelength at which absorption by hemoglobin, which is a component unique to blood, is large differences in the light absorbance are significantly expressed in blood-vessel parts and the other parts (waveform 701). Therefore, the location having large light absorbance can be easily specified. Consequently, by using a threshold value IAt determined in advance, it can be determined that a blood vessel (s) is present from X1 to X2 which is a location having larger values than the value of IAt. On the other hand, FIG. 7B is a schematic diagram of an image by the wavelength B. The wavelength B is a wavelength at which absorption to glucose is high and, at the same time, is a wavelength at which absorption by water is also high. In addition, water is present substantially evenly everywhere in a body; therefore, if detection of glucose is a purpose, the presence of water is expressed as what they call large background noise. Therefore, in the present invention, the part from X1 to X2 can be found out to be a blood vessel(s) according to the measurement by the wavelength A; therefore, the image (waveform 702) by the wavelength B can be separated into a blood-vessel part (X1 to X2) and the other part (before X1 and after X2). This contributes to precise extraction of biological information from the measurement by the wavelength B. As described above, by observing daily variations in the difference between the blood-vessel part and the other part, for example the influence, etc. of the temperature of the day which is common to the blood-vessel part and the other part can be eliminated. Moreover, when only the blood-vessel part and only the other part are separated from each other in the observation of daily variations, minute changes thereof can be easily captured.

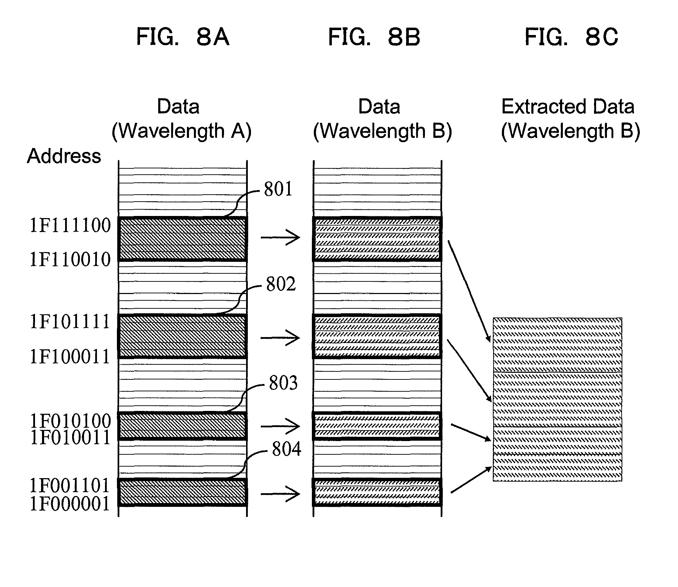

FIGS. 8A to 8C are examples in which data of the photosensor array is illustrated by address space. Data by the wavelength A is illustrated in FIG. 8A. Address regions having data that is larger than the threshold value IAt determined in advance are specified. More specifically, in FIG. 8A, the regions are the shaded parts: 1F000001 to 1F001101 (address region 801), 1F010011 to 1F010100 (address region 802), 1F100011 to 1F101111 (address region 803), and 1F110010 to 1F111100 (address region 804). In this case, the image formed on the photosensor array by the wavelength A and the image formed on the photosensor array by the wavelength B are deformed into the same address space. Therefore, in FIG. 8B of the data by the wavelength B, the data by the wavelength B in the address regions specified in relation to the data by the wavelength A is specified and extracted (FIG. 8C). When the data by the wavelength A and the data by the wavelength B is represented by the common address space in this manner, a plurality of measurement results and measurement parts can be easily connected with each other.

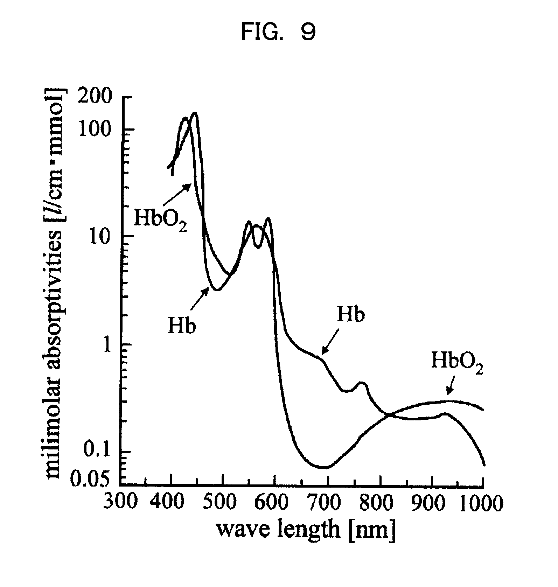

As explained in the foregoing, the wavelength at which absorption to hemoglobin, which is a component unique to blood, is high is used as the wavelength A. FIG. 9 is a diagram showing the light absorbance by hemoglobin as functions of the wavelength (Toshiyuki OZAWA, et al., "Non-Invasive Measurement of Hemoglobin Concentration in Blood by Near-Infrared Spectroscopy Image Measurement Method", Transactions of Japanese Society for Medical and Biological Engineering, vol. 43 (2005)). Hb denotes a waveform representing the data of hemoglobin in which Hb is not bound with oxygen, and HbO₂ denotes a waveform representing the data of hemoglobin bound with oxygen. As the wavelength A, a wavelength of 800 nm to 900 nm with which light absorbance by water is comparatively low and light absorbance of hemoglobin is comparatively high is used to facilitate identification of blood-vessel parts.

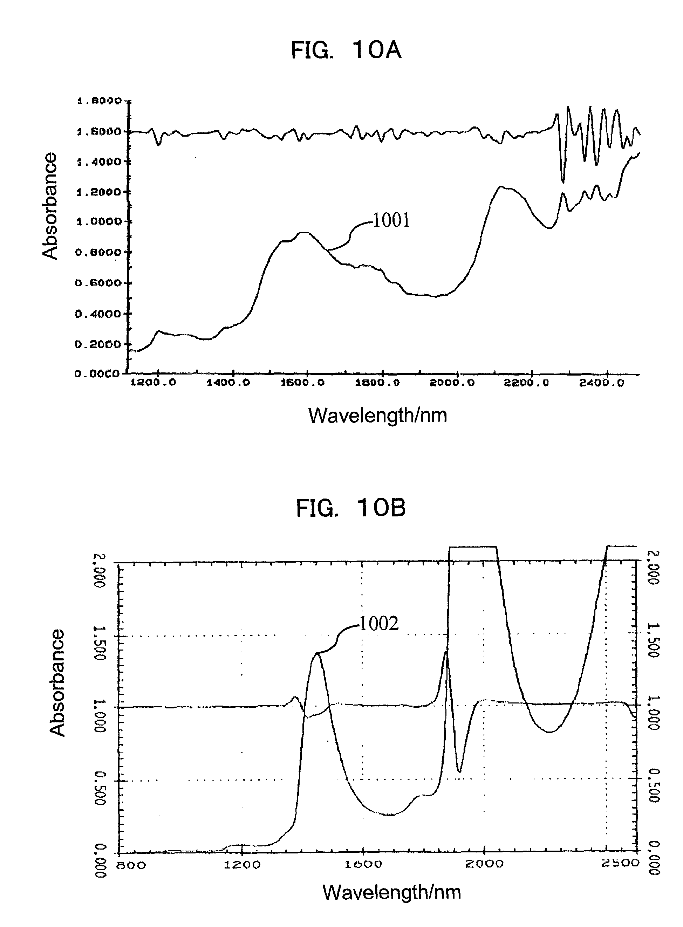

On the other hand, a wavelength at which absorption by glucose is high is used as the wavelength B. However, this wavelength region is a region in which absorption of water is also large. FIGS. 10A to 10B are diagrams showing the light absorbance of glucose (waveform 1001 of FIG. 10A) and the light absorbance of water (waveform 1002 of FIG. 10B) described in page 193 and page 211 in "Near-Infrared Spectroscopy" of Academic Publishing Center edited by Yukihiro OZAKI and Satoshi KAWATA. According to these diagrams, it can be understood that the vicinity of 1600 nm is a region in which the absorption of water is comparatively small and the absorption of glucose is large. The absorption by glucose is measured using the wavelength of this region. Furthermore, when focusing on the wavelength dependency of the light absorbance of glucose and water in a region of the wavelengths somewhat shorter than 1600 nm, for example, in the wavelength band of 1500 nm to 1600 nm, the absorbance of water is largely reduced as the wavelength becomes longer, while glucose forms a gently sloped hill of the light absorbance in this wavelength band. Therefore, when wavelengths are selected at about three points in this wavelength band and the light absorbance is measured at each of them, the data of absorbance by glucose from which the influence of absorption by water has been removed can be acquired from the differences in the changes of the light absorbance thereof.

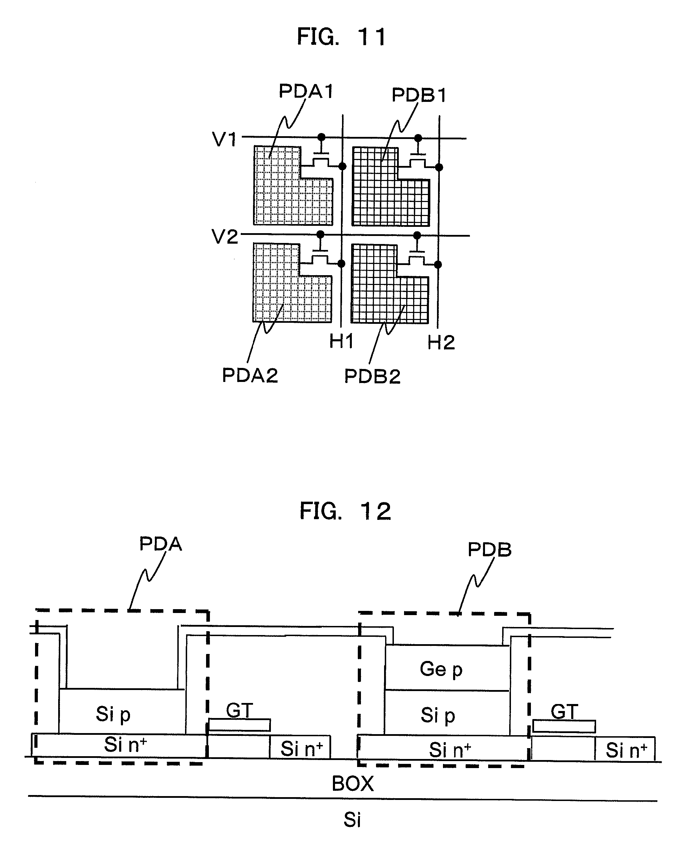

FIG. 11 is another configuration example of the photosensor array provided with dedicated photodiodes respectively for the first wavelength and the second wavelength. For example, it is carried out by the photodiode PDA, and detection of the second wavelength is carried out by the photodiode PDB. At least, the sensitivity of the photodiode PDA with respect to the first wavelength is higher than that of the photodiode PDB; on the other hand, the sensitivity of the photodiode PDB with respect to the second wavelength is higher than that of the photodiode PDA. At each wavelength, a larger difference between the sensitivities of the photodiode PDA and the photodiode PDB is preferred more. Only an array part is illustrated in this case; however, it is controlled by a peripheral circuit like that of FIG. 4. The light having the wavelength A generates electron-hole pairs in the photodiode PDA, and the signal thereof is extracted to the column signal line H1 by controlling the access transistor by the row signal line Vi. The light having the wavelength B generates electron-hole pairs in the photodiode PDB, and the signal thereof is extracted to the column signal line H2 by controlling the access transistor by the row signal line Vi. By virtue of this photosensor array, highly-sensitive measurement can be carried out for the respective wavelengths since the photodiodes for the respective wavelengths are provided. Moreover, the data by the two wavelengths can be obtained at such mutually-close locations. Therefore, if the areas of the two types of photodiodes PDA and PDB are sufficiently small with respect to the measurement region, it can be considered as the information of a single location together with the diode in the vicinity thereof. Therefore, a test region of data can be specified by the data obtained by the wavelength A, and the data obtained by the wavelength B with respect to this region can be specified. In the example of FIG. 11, the photodiodes for the same wavelength are disposed in the column direction; however, the photodiodes for the same wavelength can be configured to be disposed in the row direction.

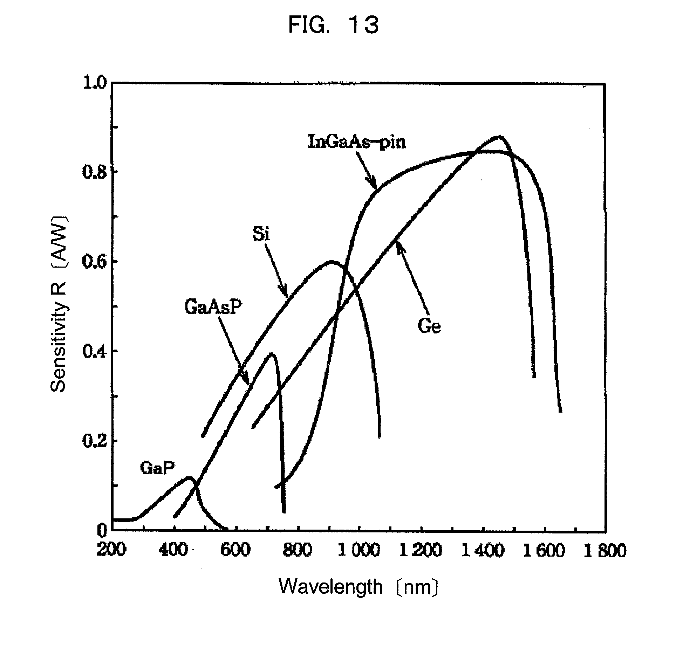

FIG. 12 is a diagram illustrating a configuration example of the photodiode PDA and the photodiode PDB. A p-type Ge layer is grown on a p-type Si layer in the photodiode PDB; on the other hand, in the photodiode PDA, no Ge layer is grown on a p-type Si layer (which is formed at the same time as the p-type Si layer of the photodiode PDB). Therefore, the photodiode PDA uses electron-hole pair generation by light of Si, and the photodiode PDB uses electron-hole pair generation by light of Ge. The band gap of Si is larger than that of Ge. Therefore, the photodiode of Si reacts against the light having a shorter wavelength than that of the photodiode of Ge. The magnitude of electron-hole pair generation with respect to the wavelengths is illustrated as sensitivity in FIG. 13 (by Takashi JIMBO, "Optical Electronics", 1997, Ohmsha, Ltd.). Si has high sensitivity against the light of about 500 to 1000 nm, and Ge has high sensitivity against the light of up to about 1550 nm. Therefore, in the photodiode PDA, the absorption of light by hemoglobin illustrated in FIG. 9 can be detected with good sensitivity; and, in the photodiode PDB, the absorption of light by glucose illustrated in FIGS. 10A and 10B can be detected with good sensitivity. Note that, although not illustrated, the photodiode PDB may be formed with mixing another material(s).

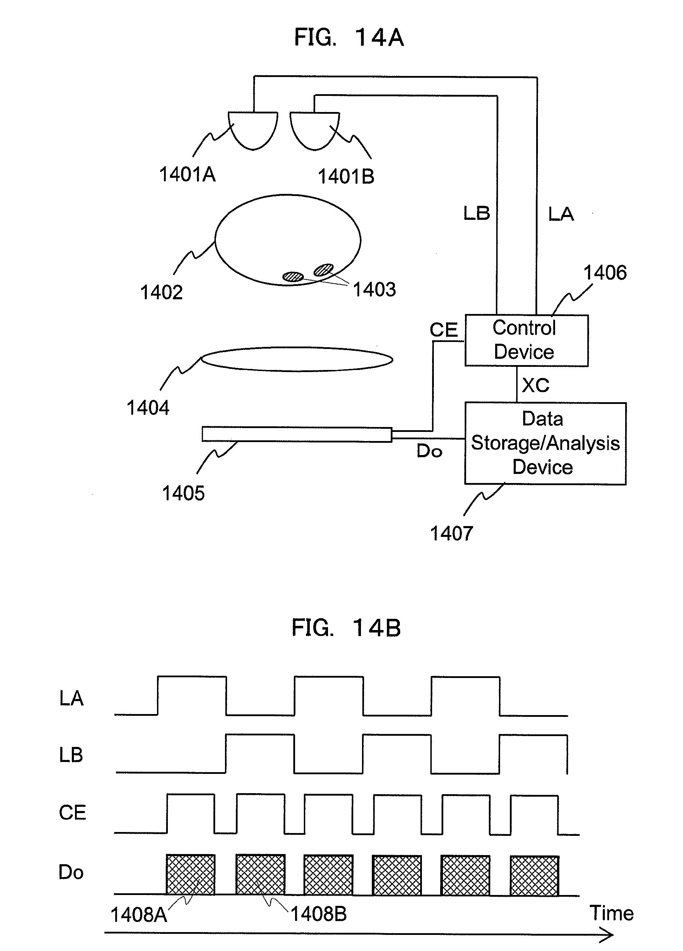

FIG. 14A is a schematic diagram illustrating a relation between a device for achieving the present invention and a finger which is a part of a human body. Two light sources are present above the finger. The light source 1401A emits light having the wavelength A, and the light source 1401B emits light having the wavelength B. A cross section of the finger 1402 irradiated by them is illustrated. A lens 1404 is present below the finger 1402, and a photosensor array 1405 is placed at a location where image formation by the lens 1404 can be obtained. The light source 1401A and the light source 1401B are controlled by control signals LA and LB from a control device 1406. As well as the control device of FIG. 5, the control device 1406 also controls the photosensor array 1405 and a data saving and controlling device 1407. The light radiated from the light source 1401 onto the finger 1402 is scattered for a plurality of times and diffused in the finger. The light that has passed through a part comparatively close to the surface of the finger is emitted to the outside. This is for a reason that attenuation of the light is kept small therein since scattering is comparatively small. Therefore, an image of veins 1403 near the surface of the finger is formed on the photosensor array 1405. FIG. 14B illustrates an operation example of the device of FIG. 14A. The control device 1406 alternately emits a signal LA, which turns on the light source 1401A, and a signal LB, which turns on the light source 1401B, as illustrated. When the light is emitted from the light source 1401A, no light is emitted from the light source 1401B. In this state, when light is emitted from the light sources, the control device 1406 activates the photosensor array 1405 by using the signal CE. The photosensor array 1405 outputs light intensity of the image as an electric signal to Do. While the light source 1401A is emitting light, data 1408A by the wavelength A is obtained; and, while the light source 1401B is emitting light, data 1408B by the wavelength B is obtained. The data 1408 is transmitted to and recorded in the data saving and analyzing device 1407, and the concentration of glucose is obtained by analysis.

Note that, when the light is alternately emitted from the two light sources, it is not necessary to obtain data from all the cells of the two-dimensional array of the photosensor array at every single light emission. For example, the cells may be divided into ten by address spaces or actual spaces, and one-tenth data may be obtained in single light emission. The data of the entire space can be obtained only one time in a series of measurement of one time; or data can be obtained for a plurality of times, and more precise analysis can be carried out by using it.

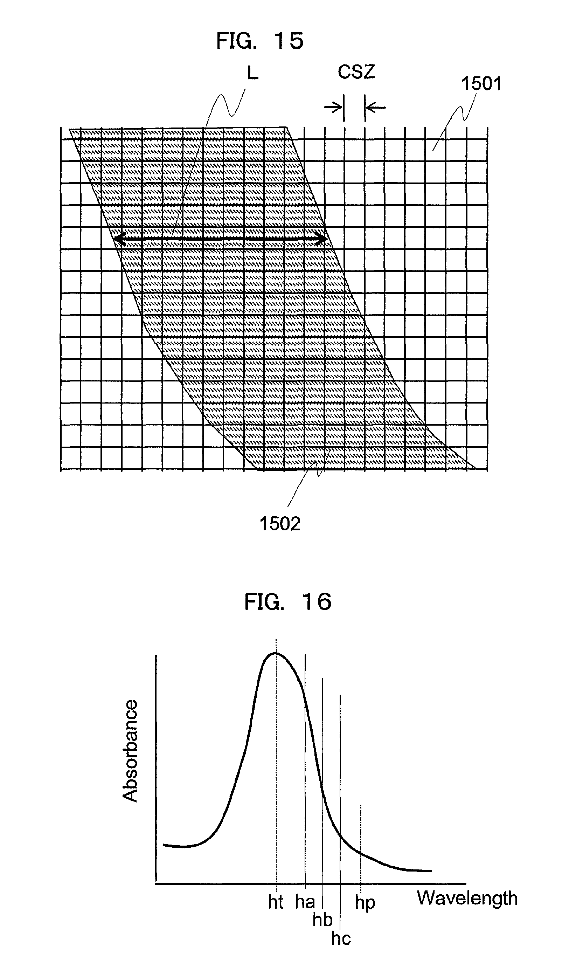

FIG. 15 is a diagram for explaining a relation between a size of the cells of the photosensor array and a diameter of a blood vessel. In this diagram, the photosensor array is expressed as a matrix 1501 having a cell size CSZ, and a state in which an image of a blood vessel 1502 is formed is illustrated. The diameter of the blood vessel is assumed to be L. It is preferable that the cells of the photosensor array have a size that at least ten cells are included in the width of the finger (CSZ < L/10). Reasons therefor will be described below. A blood-vessel part is specified as a region in which the intensity is equal to or higher than the threshold value according to the data by the wavelength A; however, the information of one to two cells on both sides of the boundary thereof is not used in many cases. The resolution power of the intensity of the single photosensor cell is about 8 bits. On the other hand, a change of glucose that causes a change in the light absorbance caused by the concentration change to be detected is only 0.1%. Therefore, at least the resolution power of 10 bits (1/2¹⁰) is required. The resolution power of 10 bits can be obtained by carrying out interpolation by the data from a plurality of cells. Also, as the diameter of a blood vessel used in measurement, it is preferable to specify a blood vessel having a diameter of 0.3 mm or more and use the information therefrom. The blood vessel of 0.3 mm or more has a stable shape, depth, etc.; therefore, the state of measurement is substantially the same every day, and more precise data of glucose to be measured can be obtained.

A modification example for improving the accuracy of the measurement will be explained. FIG. 16 illustrates wavelength dependency of light absorbance of a substance (for example, water). This example describes the maximum light absorbance at a wavelength ht, and the light absorbance is rapidly decreased therefrom toward a wavelength hp. For example, as illustrated in FIG. 10B, in the case of water, a wavelength band of 1500 nm to 1600 nm corresponds to such a part. Therefore, if the light absorbance is measured at each of ha, hb, and hc, which are wavelengths between these two wavelengths, dependency can be clearly confirmed. Particularly, if there is no dependency on the wavelengths of this region or if a substance (for example, glucose) that has dependency having a different tendency is mixed, the concentration of the substance can be obtained by separation by utilizing the wavelength dependency of the absorbance. The light having the wavelengths ha, hb, and hc can be achieved by preparing filters that selectively allow passage of the wavelengths or by using a variable wavelength laser. In order to estimate the amounts of water and glucose from the known dependencies of water and glucose and the measured dependency of a mixture of water and glucose, at least three wavelengths are required, but the measured wavelengths are not limited to three. In the case in which the filters are used, part of the wavelength band of the light source 1401B is extracted. Also in the case in which the variable wavelength laser is used, the wavelengths ha, hb, and hc can be selected from the wavelength band having a range allowed as the wavelength B as a center.

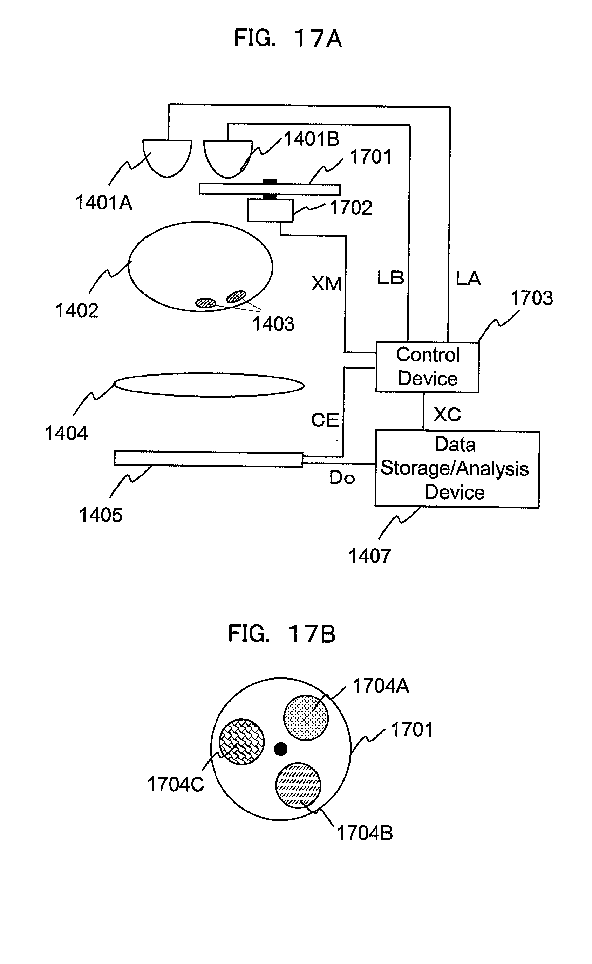

FIG. 17A illustrates a device configuration using such filters. The blocks having similar functions as those of FIGS. 14A and 14B are denoted by the same symbols. The light source 1401A is a light source for specifying the range of a measurement part, and the light source 1401B is a light source for measuring the light absorbance of a target substance; therefore, a filter plate 1701 is attached in front of the light source 1401B. In this case, the light source 1401B is a light source that emits monochromatic light; however, the wavelengths emitted therefrom have an extent. When the wavelength bands centered on ha, hb, and hc are selected from the extent so that they are not mutually overlapped, the light of the wavelengths exactly as illustrated in FIG. 16 can be obtained. The filter plate 1701 is a circular plate to which three filters (a filter 1704A, a filter 1704B, and a filter 1704C) having mutually different transparent wavelengths are attached as illustrated in FIG. 17B. This is disposed in the manner as illustrated in FIG. 17A so that it can be rotated by a motor 1702. A control device 1703 controls the motor 1702 by using a signal XM. Others are similar to the description of FIGS. 14A and 14B. The measurement using this device is also similar to the flow illustrated in FIG. 6. As the measurement of steps S603 and S604 of FIG. 6 at the wavelength B, changes are made based on the fact that measurement is carried out for three times at the wavelengths ha, hb, and hc of FIG. 16. More specifically, upon lighting of the light source 1401B, the filter plate 1701 is rotated to first irradiate the finger 1402 with the light (wavelength = ha) which has passed through the filter 1704A. Data obtained in this state is IBFAij. The filter plate 1701 is further rotated, and the finger 1402 is then irradiated with the light (wavelength = hb) which has passed through the filter 1704B. Data obtained in this state is IBFBij. The filter plate 1701 is further rotated, and the finger 1402 is irradiated with the light (wavelength = hc) which has passed through the filter 1704C. Data obtained in this state is IBFCij. The data that belongs to the photosensor cell gathering PSCA, which has been already found out, is only required to be extracted. As a result, the data by the three close wavelengths at a particular part can be obtained.

In actual measurement, the data may be acquired by sufficiently increasing the rotating speed of the filter plate controlled by the control signal XM and operating the photosensor array during irradiation only for the limited time at the speed.

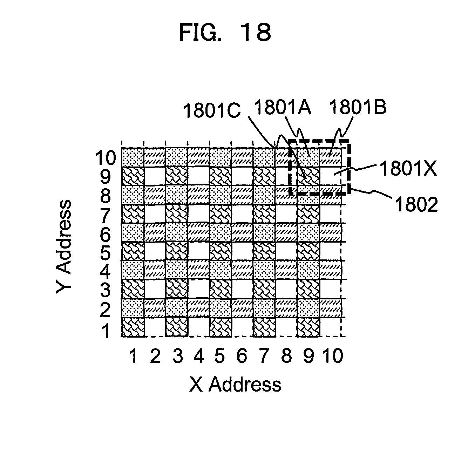

FIG. 18 is another configuration example for acquiring the data of three close wavelengths. In this example, photosensor cells are provided with filters so that only the light of specific wavelength bands is detected by the photosensor cells. In the example of the drawing, a cell 1801X with no filter is for the wavelengths A, a cell 1801A provided with a filter A is for the wavelength ha, a cell 1801B provided with a filter B is for the wavelength hb, and a cell 1801C provided with a filter C is for the wavelength hc; and a set of the cells having this four-filter layout is repeated vertically and horizontally. Such a configuration can be implemented when the size of a light receiving region of the photosensor cell is sufficiently smaller than the size of a blood vessel to be measured. Generally, down-sizing of elements advances, while the size of a blood vessel of a human is averagely constant; therefore, the data of the three close wavelengths can be acquired at one time. A location of a cell group 1801 surrounded by a dotted line is represented by the cell 1801X for the wavelength A, and the four cells can be treated to be at the same position.

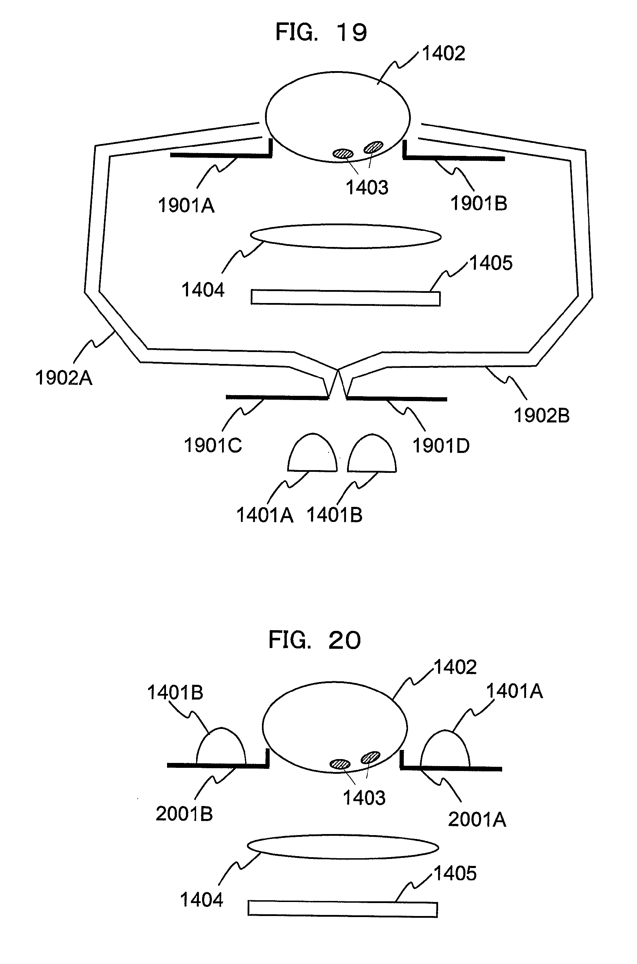

FIG. 19 is a modification example of irradiation of light from the light sources onto the finger. In this example, since the finger is not sandwiched between light sources and a photosensor array, a psychological barrier of a user can be lowered. The two light sources 1401 are placed, for example, at a lower part of the device via light shielding plates 1901. The light from the light sources is radiated from both sides of the finger 1402 via two optical fibers 1902. A lower part of the finger is exposed from a part between the light shielding plate 1901A and the light shielding plate 1901B, and the light leaked through the interior of the finger thereat forms an image on the photosensor array 1405 by the lens 1404. One-dimensionally arranged fibers or a bundle of more fibers is used as the optical fibers 1902 and irradiates the finger with light from both sides of the finger. The optical path of the optical fiber may be composed of a mirror or a prism. As illustrated in FIG. 20, the light sources may be directly disposed on both sides of the finger 1402. Light shielding plates 2001 are disposed so that the light from the light sources is not leaked to the lens 1404. According to this example, irradiation from lateral surfaces of the finger can be carried out with a simpler configuration. In the present drawing, the light source 1401A and the light source 1401B are disposed on both sides, respectively; however, a light source including the light source 1401A and the light source 1401B alternately disposed in the direction along the finger may be disposed on each of both sides of the finger.

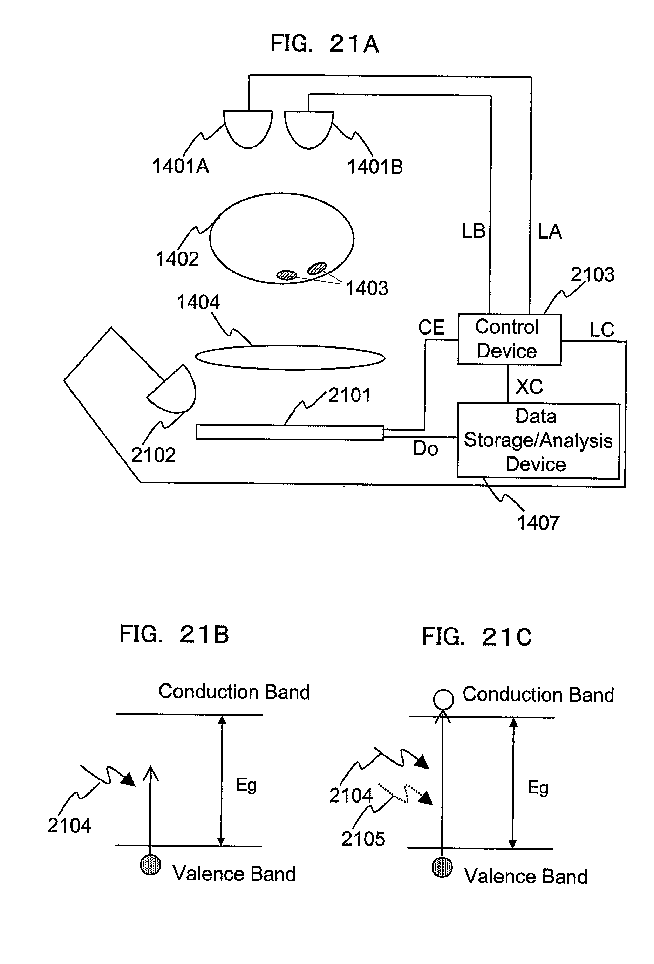

FIG. 21A illustrates a configuration example which is a still another modification example of the device and uses a low-price Si photosensor array 2101. A difference from the previous embodiments is that a light source 2102 is used, and the light source 2102 radiates the Si photosensor array. The light source 2102 is controlled by a control signal LC from a control device 2103 and is turned on in synchronization with lighting of the light source 1401B. The wavelength of the light 2105 from the light source 2102 is set so that a sum of the equivalent energy thereof and the equivalent energy of the wavelength of image forming light 2104 caused by the light from the light source 1401B is larger than 1.2 eV, which is the band gap of Si. According to this configuration, even when the low-price Si photosensor array 2102 is used, the light having a wavelength necessary for measuring the absorbance of glucose such as a wavelength of 1600 nm can be detected. The role of the light source 2102 will be explained with reference to FIGS. 21B and 21C. As illustrated in FIG. 21B, Si has a valence band and a conductive band, and an energy difference Eg called a band gap is present therebetween. The light from the light source 1401B passes through the finger and is caused to form an image on the surface of the Si photosensor array by the lens. However, even when the image forming light 2104 enters, the energy that is owned by the image forming light derived from the original light source 1401B and induces the electrons of the valence band cannot reach beyond the band gap of Si. Therefore, electron-hole pairs are not generated, and no current flows. This means that the image forming light 2104 cannot be detected. In the device of FIG. 21A, the light 2105 having a long wavelength capable of compensating for the insufficient energy is radiated from the light source 2102. In this case, the original image forming light 2104 and the light 2105 from the light source 2102 are combined, and a phenomenon of reaching beyond the band gap of Si occurs (FIG. 21C). By utilizing this phenomenon, the light absorbance of glucose like the wavelength of 1600 nm can be measured even by using the low-price Si photosensor array.

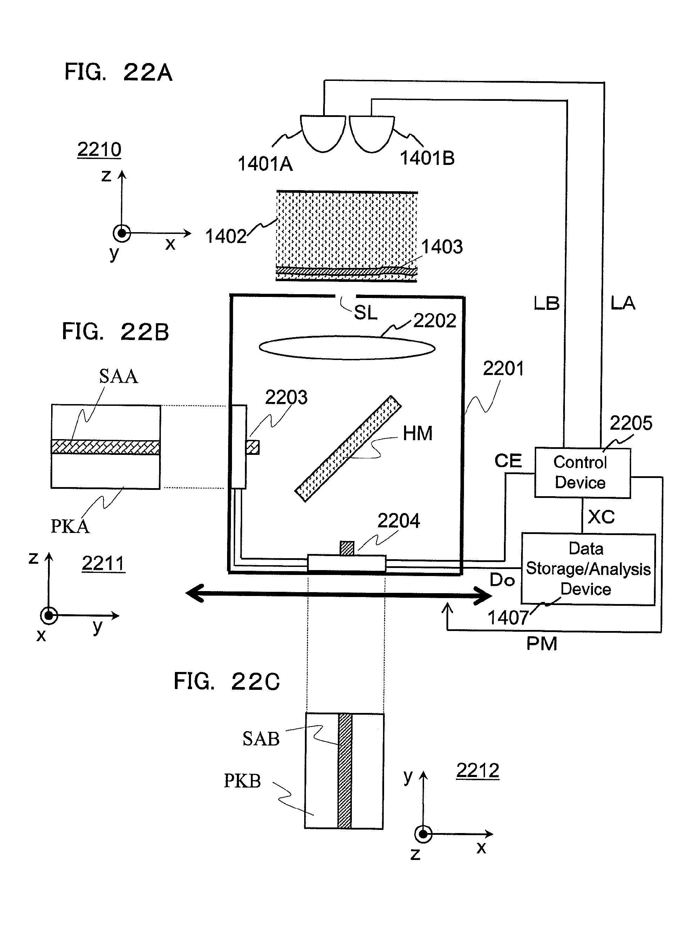

FIG. 22A is another configuration example of a biological information collecting device of the present invention, in which one-dimensional photosensor arrays are used. Two-dimensional data is equivalently acquired by scanning a certain area of a living-body tissue at the same time by the plurality of one-dimensional photosensor arrays. The light of a plurality of wavelength bands is radiated, a blood-vessel part is specified by the first wavelength, and data by the second wavelength in the region is analyzed.

The light source 1401A emits light having the wavelength A, and the light source 1401B emits the light having the wavelength B. A cross section of the finger irradiated by that is illustrated, in which the finger is placed in a state of being extended in an x direction. In the present drawing, a cross section in a vertical (pointing) direction of the finger is schematically illustrated above a chassis 2201. The light of the light source 1401A and the light source 1401B is scattered in the finger 1402, and the light emitted therefrom enters the chassis 2201 from a slit SL while mainly retaining absorption information (including the vein(s) 1403 shown in the drawing) of the skin of a part close to the chassis 2201 side.

The chassis 2201 is movable in the x direction; in the chassis 2201, at least, an optical system 2202 typified by a lens, a half mirror HM which divides light into two directions, a first one-dimensional array sensor 2203, and a second one-dimensional array sensor 2204 are disposed ahead of two optical paths formed by the half mirror HM. The first one-dimensional array sensor 2203 is, for example, a one-dimensional array sensor composed of Si photodiodes capable of detecting the light in the vicinity of 860 nm. For example, 1024 Si photodiodes are arranged in one row at intervals of 50 µm. FIG. 22B is a schematic diagram illustrating the array sensor 2203 from the upper side. The coordinate axes 2211 of FIG. 22B are for expressing a correspondence relation with the coordinate axes 2210 of FIG. 22A. The one-dimensional array sensor SAA is disposed on a supporting substrate or package PKA. On the other hand, the second one-dimensional array sensor 2204 is, for example, a one-dimensional array sensor composed of photodiodes using InGaAs capable of detecting the light in the vicinity of 1600 nm. For example, 1024 InGaAs photodiodes at intervals of 50 µm are arranged in one row. FIG. 22C is a schematic diagram illustrating the array sensor 2204 from the upper side. The coordinate axes 2212 of FIG. 22C are for expressing a correspondence relation with the coordinate axes 2210 of FIG. 22A. A one-dimensional array sensor SAB is disposed on a supporting substrate or package PKB.

The chassis 2201 can be moved in the x direction by mechanical means. This control is carried out by a signal PM from a control device 2205. The control device 2205 controls the light source 1401A by the signal LA, controls the light source 1401B by the signal LB, controls the one-dimensional array sensor 2203 and the one-dimensional array sensor 2204 by the signals CE, and controls a data saving and analyzing device by XC. The data of the one-dimensional array sensor 2203 and the one-dimensional array sensor 2204 is transmitted as Do to the data saving and analyzing device 1407. Thus, while moving the chassis 2201 by the control signal PM in a unit of a size of the hole of the slit SL, the data by the light source 1401A and the light source 1401B can be acquired on site by the one-dimensional array sensor 2203 and the one-dimensional array sensor 2204.

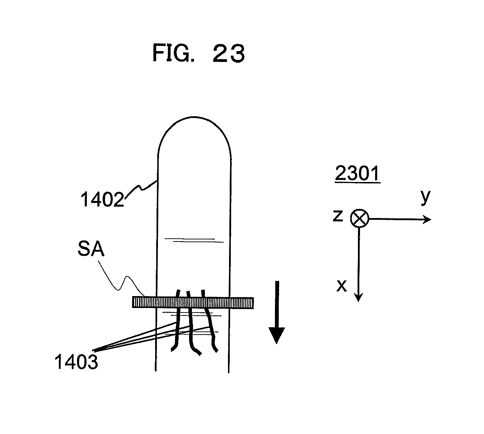

A single one-dimensional array sensor will be described with reference to FIG. 23. FIG. 23 is a diagram illustrating the state of scanning of the finger 1402 by the one-dimensional array sensor. The coordinate axes 2301 are for expressing a correspondence relation with the coordinate axes 2210 of FIG. 22A. This is a schematic diagram in which the finger is illustrated from the palm side, and the part including the back side of a nail to about two joints toward the palm is illustrated. The parts of veins which can be transparently seen by the naked eyes and can be easily checked are illustrated around the second joint from the finger tip. The pattern of the blood vessels is different for each person and is also applied in personal authentication. Small photodiodes are one-dimensionally arranged in a direction (y direction) orthogonal to the direction in which the finger is placed. Scanning is carried out by moving it a little in the x direction, then acquiring the data of scattered light using the light sources of FIGS. 22A to 22C, moving it a little again, and then similarly acquiring data, thereby equivalently obtaining two-dimensional data. The dimension of one time of scanning (the magnitude of movement of the chassis 2201) is set to the size of the slit SL.

In the device of FIGS. 22A to 22C, data is acquired by the one-dimensional array sensor 2203 and the one-dimensional array sensor 2204 by using the half mirror HM. For example, the light source 1401A and the light source 1401B are alternately blinked in one time of scanning, the light obtained when the light of the light source 1401A transmits through the finger is retrieved by the array sensor 2203, and the light obtained when the light of the light source 1401B transmits through the finger is retrieved by the array sensor 2204. Acquisition of data corresponding to this desired area is finished in several seconds. The user is only required to simply place a finger on the device including the chassis 2201 and turn on a scanning activation switch of this device. Even when the scanning activation switch is turned on, the scanning is not required to be only one time, and the scanning can be carried out for a plurality of times in order to improve measurement accuracy.

Hereinabove, the present invention has been described including a plurality of embodiments and modification examples. It goes without saying that these embodiments and modification examples can be applied in combination as long as they are not inconsistent with each other.

### Description of Reference Symbols

PD: Photodiode, AT: Access Transistor, BOX: Buried Oxide Film, Ge i : Non-Doped Germanium Layer, Ge p⁺: p-type Doped Germanium Layer, Si: Silicon Substrate, n⁺: n-type Semiconductor Region, AMP: Amplifier Circuit, DOB: Output Buffer, CE: Chip Control Signal, Do: Chip Output, XC: Data Saving And Analyzing Device Control Signal, Hb: Hemoglobin, HbO₂: Oxyhemoglobin, LA: Control Signal Of Light Source A, LB: Control Signal Of Light Source B, LC: Control Signal Of Light Source C, ht, hp, ha, hb, hc: Wavelengths

## Claims

1. A device for acquiring and analyzing biological information comprising:
a first light source radiating light having a first wavelength;
a second light source radiating light having a second wavelength;
a photosensor array detecting outgoing light from a living body irradiated with the light from the first light source and the second light source; and
an analyzer analyzing data from the photosensor array,
the analyzer specifying a region, as a blood-vessel part, in which intensity of outgoing light from the living body irradiated with the light having the first wavelength exceeds a predetermined threshold value, separating the data based on the intensity of outgoing light from the living body irradiated with the light having the second wavelength into data of the specified region and data of other regions, and subjecting the data to analysis.

2. The device for acquiring and analyzing biological information according to claim 1,
wherein the first wavelength is a wavelength between 810 nm to 940 nm; and
the second wavelength is a wavelength between 1500 nm to 1700 nm.

3. The device for acquiring and analyzing biological information according to claim 1,
wherein the light having the first wavelength and the second wavelength is near infrared light;
the first wavelength is in a wavelength band in which absorption by hemoglobin is relatively high; and
the second wavelength is in a wavelength band in which absorption by glucose is relatively high.

4. The device for acquiring and analyzing biological information according to claim 1,
wherein the intensity of the outgoing light from the living body irradiated with the light having the second wavelength depends on at least amounts of a first substance and a second substance;
the device has a mechanism that radiates light having a plurality of wavelengths from a wavelength band centered on a range allowable as the second wavelength;
the analyzer obtains dependency of the intensity of the outgoing light and a radiated wavelength from intensity data of the outgoing light from the living body irradiated with the light having the plurality of wavelengths; and
the first substance and the second substance contained in the blood-vessel part are separated from each other based on the dependency.

5. The device for acquiring and analyzing biological information according to claim 4, comprising,
as the mechanism, a plurality of filters each allowing passage of light of a predetermined wavelength band from the second light source.

6. The device for acquiring and analyzing biological information according to claim 1,
wherein, in the photosensor array, photosensor cells are arranged in a two-dimensional matrix;
the photosensor cells each include a first photosensor cell that has a first diode and a second photosensor cell that has a second photodiode;
the first photosensor cell and the second photosensor cell are arranged to be mutually adjacent in the photosensor array;
the first photodiode has higher sensitivity to the first wavelength compared with that of the second photodiode; and
the second photodiode has higher sensitivity to the second wavelength compared with that of the second photodiode.

7. The device for acquiring and analyzing biological information according to claim 6,
wherein the first photodiode uses electron-hole pair generation by Si caused by light; and
the second photodiode uses electron-hole pair generation by Ge caused by light.

8. The device for acquiring and analyzing biological information according to claim 1, comprising
a mechanism that radiates the light of the light source from a lateral surface of the living body.

9. The device for acquiring and analyzing biological information according to claim 1, further comprising
a third light source radiating light having a third wavelength,
wherein the photosensor array is composed of a photosensor cell having a photodiode using electron-hole pair generation by Si caused by light; and
the third light source emits light in synchronization with the second light source.

10. The device for acquiring and analyzing biological information according to claim 1,
wherein the photosensor array has a first photosensor array and a second photosensor array in which photosensor cells are one-dimensionally arranged;
the first photosensor array has a first photosensor cell having a first photodiode;
the second photosensor array has a second photosensor cell having a second photodiode;
the first photodiode has higher sensitivity to the first wavelength compared with that of the second photodiode;
the second photodiode has higher sensitivity to the second wavelength compared with that of the second photodiode; and
the first and second photosensor arrays are caused to scan a predetermined range of the living body.

11. The device for acquiring and analyzing biological information according to claim 10, comprising
a half mirror,
the half mirror causing the light having the first wavelength to be received by the first photosensor array and causing the light having the second wavelength to be received by the second photosensor array.
